# EUROPEAN PATENT SPECIFICATION

(11) **EP 4 232 018 B1**
(45) Date of publication and mention of the grant of the patent: **20.08.2025**
(21) Application number: 21802003.0
(22) Date of filing: 21.10.2021
(51) Int. Cl.: A61K 31/24, A61K 31/216, A61P 31/12, A61P 31/14

(54) **COMPOUNDS FOR USE IN TREATING CORONAVIRUS INFECTION AND AFRICAN SWINE FEVER VIRUS INFECTION**
VERBINDUNGEN ZUR BEHANDLUNG VON CORONAVIRUS-INFEKTIONEN UND AFRIKANISCHEN SCHWEINEPEST VIRUS-INFEKTIONEN
COMPOSÉS UTILISÉS DANS LE TRAITEMENT D'UNE INFECTION CAUSÉE PAR UN CORONAVIRUS ET PAR LE VIRUS DE LA PESTE PORCINE AFRICAINE

(30) Priority: 21.10.2020 GB 202016697
(43) Date of publication of application: 30.08.2023
(73) Proprietor: RHEA GENETICS PTE. LTD., Singapore 536204 (SG)
(72) Inventor: JIARAVANON, Benjamin, Singapore 536204 (SG); BABIKIAN, Haig, Singapore 536204 (SG)
(74) Representative: Reddie & Grose LLP
(86) International application number: PCT/IB2021/059725
(87) International publication number: WO 2022/084916

(56) References cited:
- WO-A1-2018/132066
- WO-A1-2020/212901
- GUMENIUK M.I. ET AL: "Efficacy of decamethoxin against complex viruses regardless of their antigenic structure: prospects for use in modern viral diseases of the respiratory tract", ACTUAL INFECTOLOGY, vol. 8, no. 1, 1 January 2020 (2020-01-01), pages 25 - 33, XP055880425, ISSN: 2312-413X, DOI: 10.22141/2312-413x.8.1.2020.196168

## Description

The present invention relates to a compound and composition for use in treating an infection caused by a coronavirus or African Swine fever virus.

Coronaviruses are a group of RNA viruses that cause diseases in mammals and birds. Typically, in humans these viruses cause respiratory tract infections. In humans, some coronaviruses can be responsible for the common cold as well as more serious diseases such as Severe acute respiratory syndrome (SARS) and Coronavirus disease 2019 (COVID-19).

In 2019, a novel coronavirus known as severe acute respiratory syndrome coronavirus 2 (SARS-CoV-2) emerged causing coronavirus disease 2019 (COVID-19). Covid-19 quickly spread across the planet causing a global pandemic. The severity of COVID-19 varies from mild through to lethal. Risk factors include underlying health conditions, old age and obesity. Cases of symptoms lingering for many months after initial recovery from the disease (so called long Covid-19) have also been reported. The virus is particularly contagious and rapidly spread throughout the world during late 2019 and 2020 causing significant morbidity and economic disruption.

Given the severity of Covid-19 and the ease of transmission of SARS-CoV-2, there is a need for new antiviral treatments for Covid-19.

African Swine Fever is a disease that causes a high mortality rate in domestic pigs. The disease is caused by the African Swine Fever virus (ASFV) which is a large double stranded DNA virus in the Asfarviridae family. The disease can be devastating to domestic pig populations. There is a need for new treatments to address this disease.

WO 2018/132066 relates to compounds for use to treat Herpesviradae Human Papilloma Virus, bacterial infections and fungal infections.

GUMENIUK et al. (2020)¹, relates to the antiviral activity of decamethoxin regardless of antigenetic structure, giving grounds for use of decamethoxin in respiratory tract infections, including coronavirus infection.

¹ GUMENIUK M.I et al., Efficacy of decamethoxin against complex viruses regardless of their antigenic structure: prospects for use in modern viral disease of the respiratory tract, Actual Infectology, January 2020, vol. 8, no. 1, abstract.

In a first aspect of the present invention, there is provided a compound, or a composition comprising the compound, for use in the treatment of an infection caused by a coronavirus or African swine fever virus; wherein the compound is a compound of formula I: 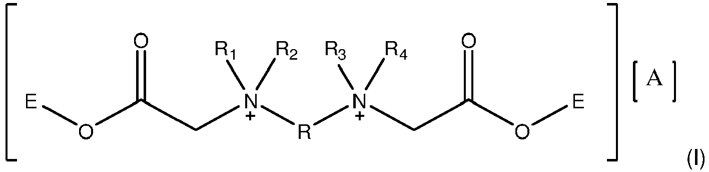
wherein R is an alkylene chain having between 8 and 20 carbon atoms, and A is one or more anions having a total charge of -2,
or R is a group having the following formula: 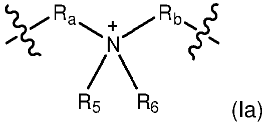
wherein Rₐ and R_{b} are each an alkylene chain having between 8 and 20 carbon atoms, and A is one or more anions having a total charge of -3;
R₁, R₂, R₃, R₄, R₅ and R₆ are each independently selected from C₁₋₁₀ alkyl and H;
E has the following formula 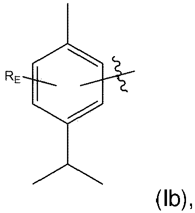 wherein the ester oxygen is bonded to the aromatic ring of each E at the same position, the position being position 2 or 3; and wherein R_{E} is H or a halide.

In the case that the compound (or the composition comprising the compound) is for use in treating an infection caused by a coronavirus, the compound or composition may be for use in treating an infection caused by a severe acute respiratory syndrome coronavirus, for example severe acute respiratory syndrome coronavirus 2 (SARS-CoV-2).

The compound (or the composition comprising the compound) may be for use in treating a coronavirus disease, for example coronavirus disease 2019 (COVID-19).

The compound (or the composition comprising the compound) may be for use in treating an infection caused by African swine fever virus, for example African swine fever.

In the compound of formula (I) R may be, for example, a linear or branched saturated or unsaturated alkylene chain having between 8 and 18 carbon atoms, for example 8 and 16 carbon atoms, for example 8 and 14 carbon atoms, for example 9 and 15 carbon atoms, for example 10 carbon atoms. Preferably, R is a linear saturated alkylene chain having between 8 and 16 carbons, for example 10 carbon atoms. Herein alkylene means an alkane based di-radical, so has two points of attachment to the rest of the molecule.

R may be, for example, a group according to formula (la), in which Rₐ and R_{b} are each a linear or branched saturated or unsaturated alkylene chain having between 8 and 18 carbon atoms, for example 8 and 16 carbon atoms, for example 8 and 14 carbon atoms, for example 9 and 15 carbon atoms, for example 10 carbon atoms. Rₐ and R_{b} may each be a linear or branched saturated alkylene chain having a different number of carbon atoms or the same number of carbon atoms.

Preferably, Rₐ and R_{b} are each a linear saturated alkylene chain having between 8 and 16 carbon atoms, for example 10 carbon atoms.

A saturated linear alkylene chain may be represented by the following formula: 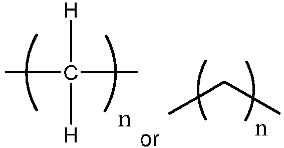 wherein n is the number of repeat units, that is the number of carbon atoms in the linear alkylene chain. Thus, in the case of R being an alkylene chain having between 8 and 20 carbon atoms, it is preferred that R is 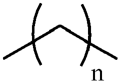 wherein n is between 8 and 16, for example 10. In the case of R being an amine having the formula (la) as set out above, it is preferred that Rₐ and R_{b} are each 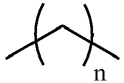 wherein n is between 8 and 16, for example 10.

In the case of, for example, R being an alkylene chain having 10 carbons, R may be represented by the following formula

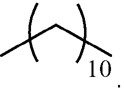

A may, for example, comprise halide ions, such as chloride (CI) ions, bromide (Br⁻) ions, iodide ions (I⁻) and/or fluoride ions (F⁻). A may, for example, comprise ions of other organic and non-organic acids, such as sulphate (SO₄²⁻), carbonate (CO₃²⁻), hydrogen carbonate (HCO₃⁻), hydrogen sulphate (HSO₄⁻), acetate ions (CH₃COO⁻), and/or formate ions (HCOO⁻). In the case of R being an alkylene chain having between 8 and 20 carbon atoms, preferably A comprises two halide ions, for example two chloride ions, thus having a total charge of -2. In the case of R being a group having formula (la) as set out above, preferably A comprises two chloride ions and a bromide ion, thus having a total charge of -3.

R₁, R₂, R₃, R₄, R₅ and R₆ may each independently be selected from C₁₋₄ alkyl and H, for example R₁, R₂, R₃, R₄, R₅ and R₆ may each be methyl. R_{E} may be chloride, bromide, iodide, or fluoride. In the case of the ester oxygen being bonded to the aromatic ring of E at position 2 with respect to the methyl group, R_{E} may be bonded to the aromatic ring at position 3, 5 or 6 with respect to the methyl group. In the case of the ester oxygen being bonded to the aromatic group of E at position 3 with respect to the methyl group, R_{E} may be bonded to the aromatic ring at position 2, 5 or 6 with respect to the methyl group.

Preferably, the compound of formula I above has the following formula: 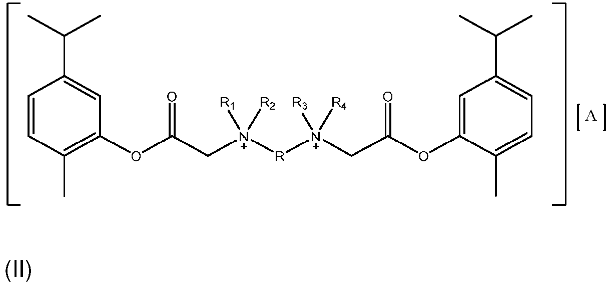 or 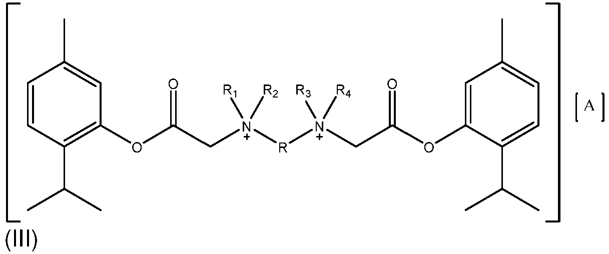 in which R, R₁, R₂, R₃, R₄ and A are as defined above for formula I.

Preferably, R₁, R₂, R₃, R₄, R₅ and R₆ are each methyl.

The compound of formula I may have the following formula: 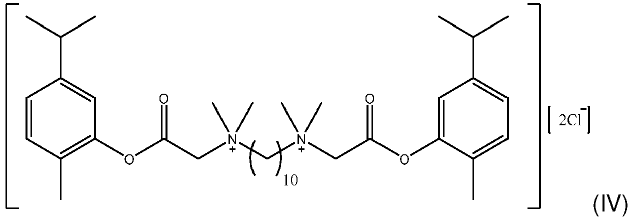 or 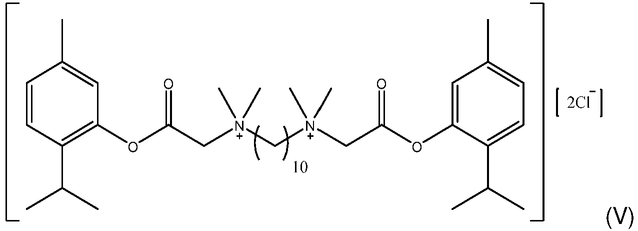 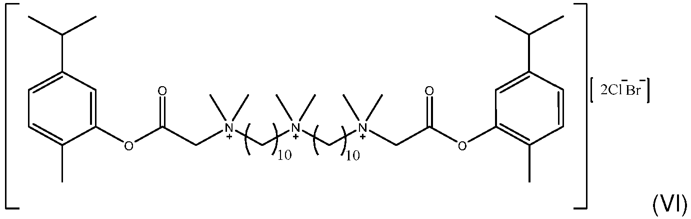 or

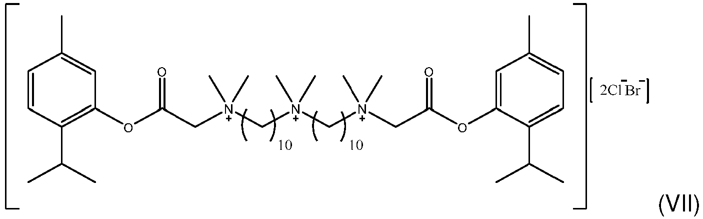

Preferably, the compound of formula I has the following formula: 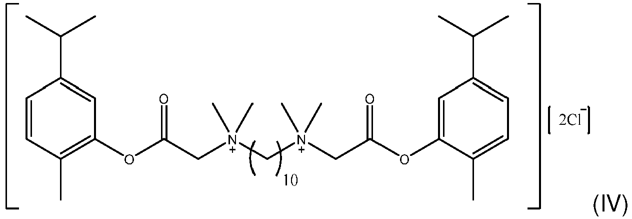 or

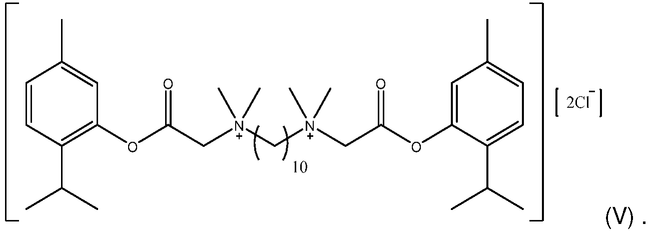

In formula IV and V, it will be appreciated that R is a linear saturated alkylene chain having 10 carbon atoms, and A is two chloride ions. In formula VI and VII, it will be appreciated that R is a quaternary amine, in which Rₐ and R_{b} are each saturated linear alkylene chains having 10 carbon atoms, and A is two chloride ions and one bromide ion. It will be appreciated that in formula IV and V, R₁, R₂, R₃ and R₄ are each methyl. It will be appreciated that in formula VI and VII, R₁, R₂, R₃, R₄, R₅ and R₆ are each methyl.

In the case in which the compound of formula I (or the composition comprising the compound of formula I) is for use in treating an infection caused by a severe acute respiratory syndrome coronavirus, for example caused by Severe acute respiratory syndrome coronavirus 2 (SARS-CoV-2), or in the case in which the compound of formula I (or the composition comprising the compound of formula I) may be for use in treating a coronavirus disease, for example coronavirus disease 2019 (COVID-19), then preferably the compound of formula I has the following formula: 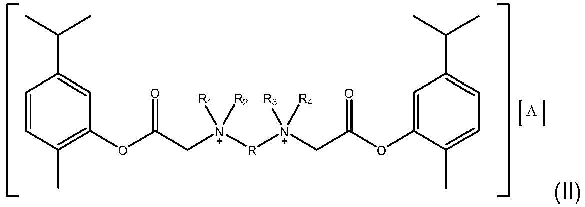 wherein R is an alkylene chain having between 8 and 20 carbon atoms, for example R may be a saturated linear alkylene chain having between 8 and 16 carbon atoms, for example 10 carbon atoms.

The compound of formula II may have the following formula:

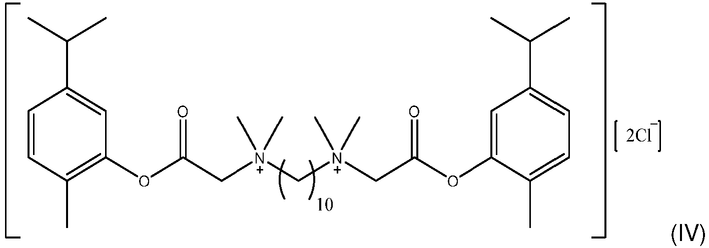

In the case in which the compound of formula I (or the composition comprising the compound of formula I) is for use in treating an infection caused by African Swine Fever virus, for example for use in treating African Swine Fever, then preferably the compound of formula I has the following formula: 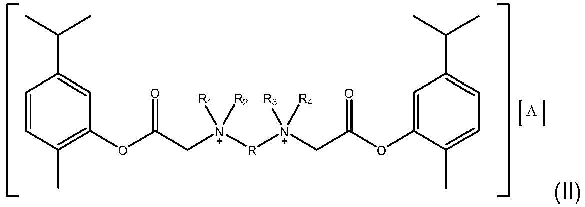 or 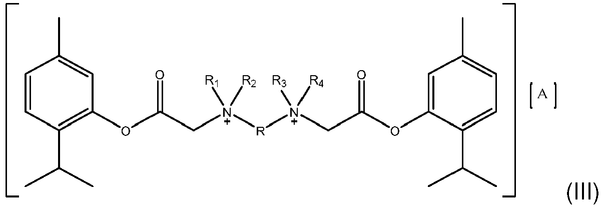 wherein R is an alkylene chain having between 8 and 20 carbon atoms, for example R may be a saturated linear alkylene chain having between 8 and 16 carbon atoms, for example 10 carbon atoms.

The compound of formula II may have the following formula:

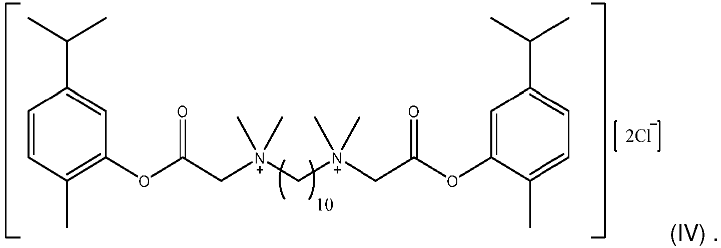

The compound of formula III may have the following formula:

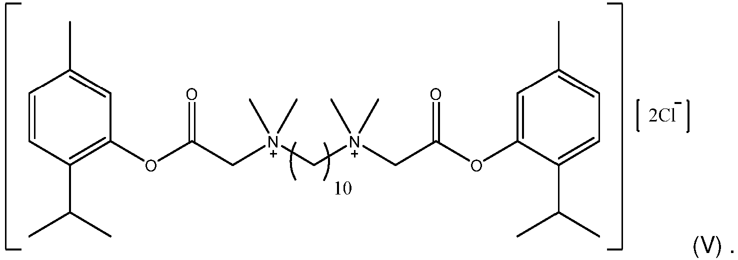

In other embodiments, the compound may be a compound of formula VIII 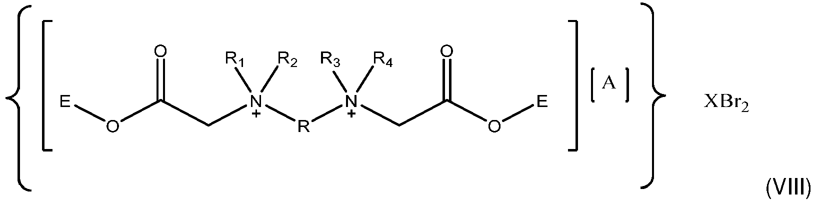
wherein R is an alkylene chain having between 8 and 20 carbon atoms, and A is one or more anions having a total charge of -2,
or R is a group having the following formula: 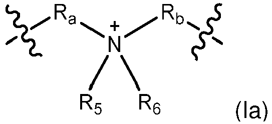
wherein Rₐ and R_{b} are each an alkylene chain having between 8 and 20 carbon atoms, and A is one or more anions having a total charge of -3;
R₁, R₂, R₃, R₄, R₅ and R₆ are each independently selected from C₁₋₁₀ alkyl and H;
E has the following formula 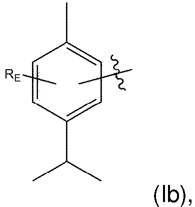 wherein the ester oxygen is bonded to the aromatic ring of each E at the same position, the position being position 2 or 3; wherein R_{E} is H or a halide; and wherein X is 2, 3, 4 or 6.

Each of R, R₂, R₃, R₄, R₅ and R₆, Rₐ, R_{b}, and A may be as defined according to any statement above.

Preferably, the compound of formula VIII above has the following formula: 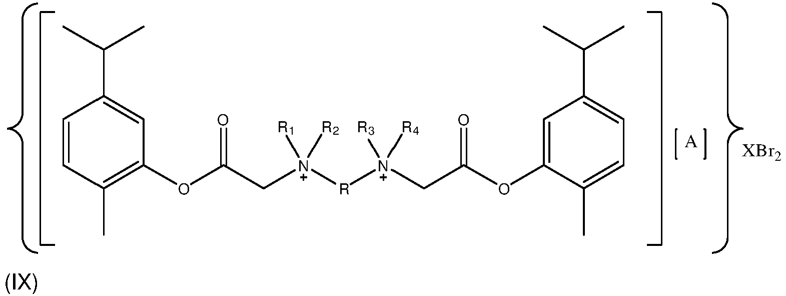 or 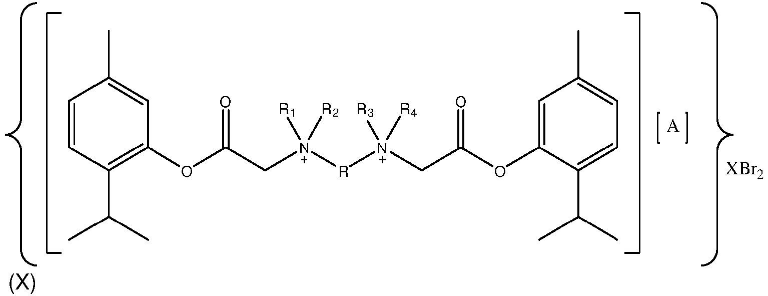 wherein R, R₁, R₂, R₃, R₄, X and A are as defined above for formula (IX).

Preferably, the compound of formula (VIII) has the following formula: 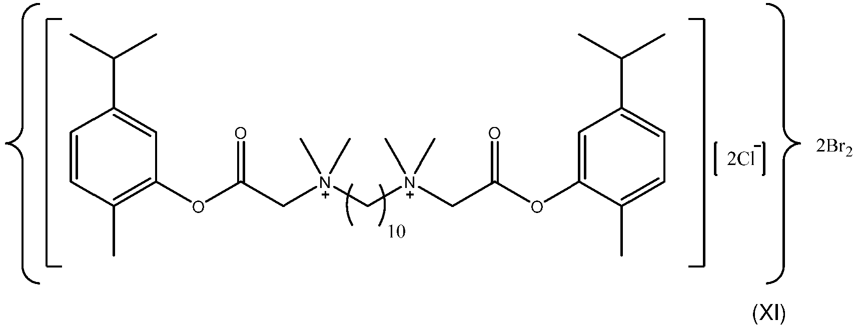 or 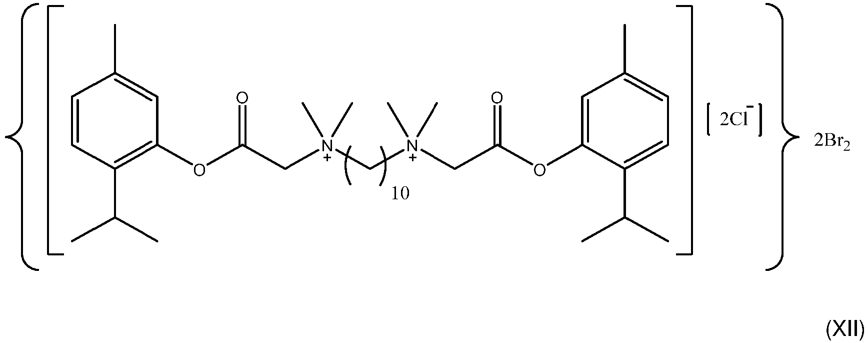 or 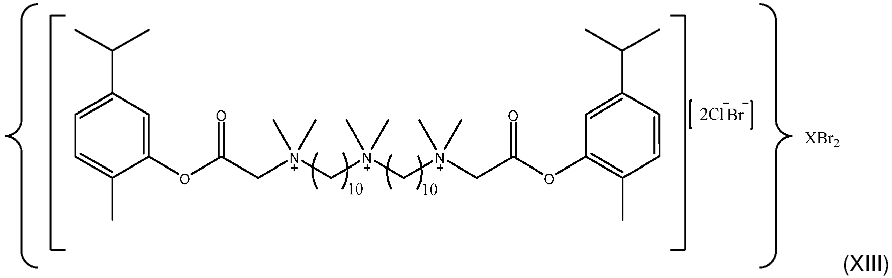 or 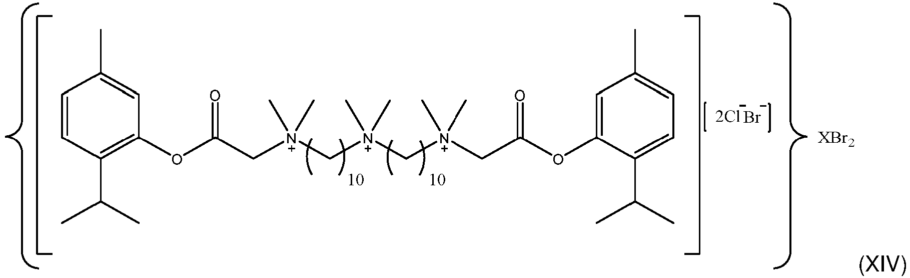 wherein X is 3 or 6.

In formula XI and XII, it will be appreciated that R is a linear saturated alkylene chain having 10 carbon atoms; and A is two chloride ions.

In formula XIII and XIV, it will be appreciated that R is a quaternary ammonium in which Rₐ and R_{b} are each saturated linear alkylene chains having 10 carbon atoms, and A is two chloride ions and one bromide ion. It will be appreciated that in formula XI and XII, R₁, R₂, R₃ and R₄ are each methyl. It will be appreciated that in formula XIII and XIV, R₁, R₂, R₃, R₄, R₅ and R₆ are each methyl.

The composition may be a pharmaceutical composition, for example a human pharmaceutical composition and/or a veterinary pharmaceutical composition.

The pharmaceutical composition may be in a form suitable for one or more of oral, rectal, parenteral, transdermal, intravenous, intra-arterial, intraosseous infusion, intracerebral, intracerebroventricular, intrathecal, intramuscular, subcutaneous, intravaginal, intraperitoneal, epidural, intracerebral, intraosseous infusion, intravitreal, transmucosal, buccal, or nasal administration. Preferably, the pharmaceutical composition is in the form suitable for oral or intravenous administration.

The pharmaceutical composition may further comprise a pharmaceutically acceptable carrier, such as aqueous solution, non-toxic excipients, including salts and preservatives, or buffers.

Examples of suitable aqueous and non-aqueous pharmaceutical carriers, diluents, solvents or vehicles include water, ethanol, polyols (such as glycerol, propylene glycol, polyethylene glycol, and the like), carboxymethylcellulose and suitable mixtures thereof, vegetable oils (such as olive oil), and injectable organic esters such as ethyl oleate.

Examples of pharmaceutically acceptable excipients include antiadherents, binders, coatings, colourings, disintegrant, flavourings, glidants, lubricants, preservatives, sorbents and sweeteners.

The pharmaceutical composition may also contain additives such as but not limited to preservatives, wetting agents, emulsifying agents, surfactants and dispersing agents. Antibacterial and antifungal agents can be included to prevent growth of microbes and includes, for example, m-cresol, benzyl alcohol, paraben, chlorobutanol, phenol, sorbic acid, and the like. If a preservative is included, benzyl alcohol, phenol and/or m-cresol are preferred; however, the preservative is by no means limited to these examples. Furthermore, it may be desirable to include isotonic agents such as sugars, or sodium chloride.

A pharmaceutical composition suitable for oral administration may be in the form of, for example, a tablet, a pill, a sugar coated agent, a powder, a capsule, a liquid, a gel, a syrup, a slurry, a suspension, a cachet and the like. The composition may comprise a pharmaceutically acceptable carrier, for example liposomes, lactose, trehalose, sucrose, mannitol, xylitol, crystalline cellulose, chitosan, calcium carbonate, talc, titanium oxide, or silica.

The pharmaceutical composition may be obtained, for example, by combining the compounds of the invention with a solid excipient, pulverizing the mixture (if necessary) and inserting into a capsule, for example, a soft sealed capsule consisting of a gelatin capsule, gelatin and coating (e.g., glycerol or sorbitol) or a capsule composition suitable for vegetarians. In the soft capsule, the composition may be dissolved or suspended in an appropriate liquid, such as a fatty oil, liquid paraffin or liquid polyethylene glycol, with or without a stabilizer.

The compound or composition may be administered according to one or more of the following administration routes: oral, rectal, parenteral, transdermal, intravenous, intra-arterial, intraosseous infusion, intracerebral, intracerebroventricular, intrathecal, intramuscular, subcutaneous, intravaginal, intraperitoneal, epidural, intracerebral, intraosseous infusion, intravitreal, transmucosal, buccal, or nasal. Preferably, the compound or composition is administered orally or intravenously.

The compound may be administered in a dose of from 0.5 to 10 µg per kg of bodyweight.

The present invention will now be described in more detail with reference to the following examples and the accompanying drawings in which:
Figure 1 illustrates the effect of compound IV on inhibition of the SARS-CoV-2 reporter gene; and
Figure 2 indicates the effect of compound IV on cell viability.

### Examples

### Example 1

### Synthesis of the compound of formula (IV).

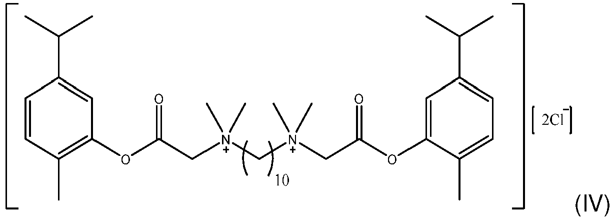

In a first step, 1,10-dibromodecane is reacted with 2 molar equivalents of dimethylamine to form 1,10-Bis(dimethylamino)decane. The reaction takes place at 4-5° C in a suitable solvent such as benzene, diethyl ether or dimethylamine and is followed by a step of acid extraction followed by alkaline treatment and extraction with diethyl ether. The extracted fractions are dried over magnesium sulphate and then purified by vacuum distillation.

In a second step, Carvacrol (2-Methyl-5-(1-methylethyl)-phenol) is reacted with chloroacetyl chloride. The reaction is carried out at -10° C for 1 hour and then stirred at room temperature for 12 hours. The reaction mixture is then washed with acid, followed by treatment with sodium bicarbonate and then water. The organic layer is dried over sodium sulphate, filtered and the solvent is removed under vacuum.

In a third step, 2 molar equivalents of the compound formed in the second step is reacted with one molar equivalent of 1,10-Bis(dimethylamino)decane to form the compound of formula (IV). The reaction in the third step is carried out using an appropriate solvent such as acetonitrile and the reaction mixture is stirred for 24 hours.

It will be appreciated that further purification and separation steps may also be included in the process, for example between each of the above steps and also after the process is complete to purify the final compound having formula (IV).

Separation steps may include steps of performing column chromatography, low pressure liquid chromatography, high performance liquid chromatography and the like. Purification steps may include standard purification processes known in the art, for example, filtration, evaporation, liquid-liquid extraction, crystallisation, adsorption, recrystallization, chromatography, distillation and the like.

The compound of formula (IV) has the following systematic name, formula and molecular weight:

| | |
|---|---|
| Systematic name: | N1,N10-bis(2-(5-isopropyl-2-methylphenoxy)-2-oxoethyl)-N1,N1,N10,N10-tetramethyldecane-1,10-diaminium dichloride |
| Formula: | C₃₈H₆₂N₂O₄⁺² 2Cl⁻ |
| Molecular weight: | 681.81 |

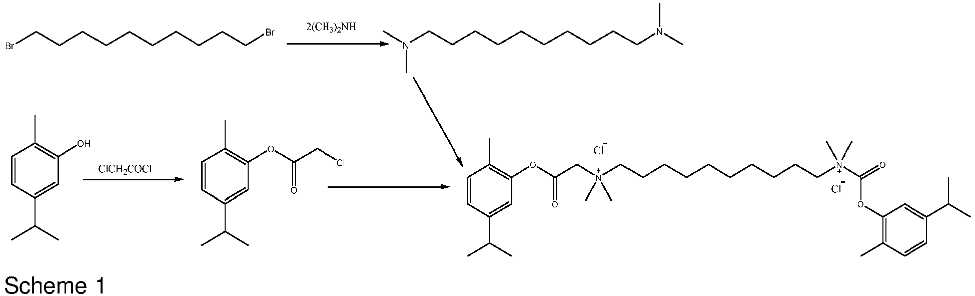

### Example 2

### Synthesis of the compound of formula (V)

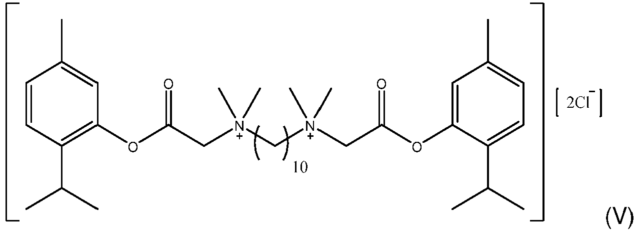

In a first step, 1,10-dibromodecane is reacted with 2 molar equivalents of dimethylamine to form 1,10-Bis(dimethylamino)decane. The reaction takes place at 4-5° C in benzene in a suitable solvent such as benzene, diethyl ether or dimethylamine and is followed by a step of acid extraction followed by alkaline treatment and extraction with diethyl ether. The extracted fractions are dried over magnesium sulphate and then purified by vacuum distillation.

In a second step, thymol (2-isopropyl-5-methylphenol) is reacted with chloroacetyl chloride. The reaction is carried out at -10° C for 1 hour and then stirred at room temperature for 12 hours. The reaction mixture is then washed with acid, followed by treatment with sodium bicarbonate and then water. The organic layer is dried over sodium sulphate, filtered and the solvent is removed under vacuum.

In a third step, 2 molar equivalents of the compound formed in the second step is reacted with one molar equivalent of 1,10-Bis(dimethylamino)decane to form the compound of formula (V). The reaction in the third step is carried out using an appropriate solvent such as acetonitrile and the reaction mixture is stirred for 24 hours.

It will be appreciated that further purification and separation steps may also be included in the process, for example between each of the above steps and also after the process is complete to purify the final compound having formula (V).

Separation steps may include steps of performing column chromatography, low pressure liquid chromatography, high performance liquid chromatography and the like. Purification steps may include standard purification processes known in the art, for example, filtration, evaporation, liquid-liquid extraction, crystallisation, adsorption, recrystallization, chromatography, distillation and the like.

| | |
|---|---|
| Systematic Name: | N1,N10-bis(2-(2-isopropyl-5-methylphenoxy)-2-oxoethyl)-N1,N1,N10,N10-tetramethyldecane-1,10-diaminium dichloride |
| Formula: | C₃₈H₆₂N2O₄⁺² 2Cl- |
| Molecular Weight: | 681.81 |

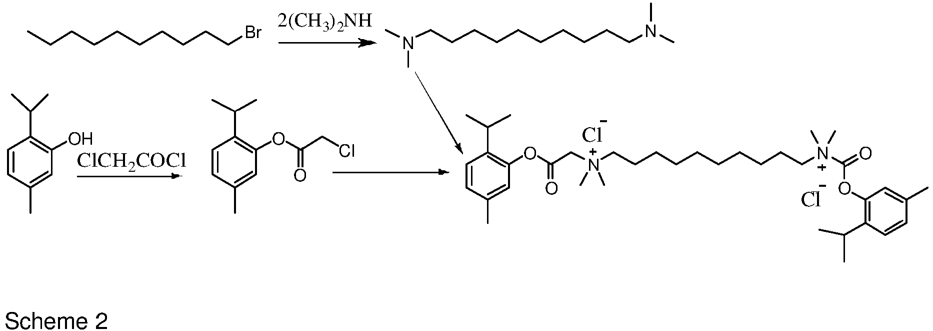

### Example 3

### Synthesis of the compound of formula (VI)

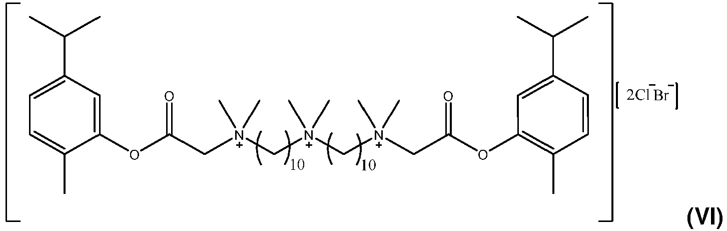

In a first step, 2 molar equivalents of 1,10-Dibromodecane are reacted with 3 molar equivalents of dimethylamine. The reaction takes place at 4-5° C in a suitable solvent such as benzene or diethyl ether and is followed by a step of acid extraction followed by alkaline treatment and extraction with diethyl ether. The extracted fractions are dried over magnesium sulphate and then purified by vacuum distillation.

In a second step, carvacrol (2-Methyl-5-(1-methylethyl)-phenol) is reacted with chloroacetyl chloride. The reaction is carried out at -10 C for 1 hour and then stirred at room temperature for 12 hours. The reaction mixture is then washed with acid, followed by treatment with sodium bicarbonate and then water. The organic layer is dried over sodium sulphate, filtered and the solvent is removed under vacuum.

In a third step, one molar equivalent of the compound formed in step 1 is reacted with the 2 molar equivalents of the compound formed in step 2 to form the compound having formula (VI). The reaction in the third step is carried out using an appropriate solvent such as acetonitrile and the reaction mixture is stirred for 24 hours.

It will be appreciated that further purification and separation steps may also be included in the process, for example between each of the above steps and also after the process is complete to purify the final compound having formula (VI).

Separation steps may include steps of performing column chromatography, low pressure liquid chromatography, high performance liquid chromatography and the like. Purification steps may include standard purification processes known in the art, for example, filtration, evaporation, liquid-liquid extraction, crystallisation, adsorption, recrystallization, chromatography, distillation and the like.

| | |
|---|---|
| Systematic Name: | N1-(2-(5-isopropyl-2-methylphenoxy)-2-oxoethyl)-N10-(10-((2-(5-isopropyl-2-methylphenoxy)-2-oxoethyl)dimethylammonio)decyl)-N1,N1,N10,N10-tetramethyldecane-1,10-diaminium bromide dichloride |
| Formula: | C₅₀H₈₈N₃O₄⁺³ Br⁻2Cl⁻ |
| Molecular Weight: | 946.06 |

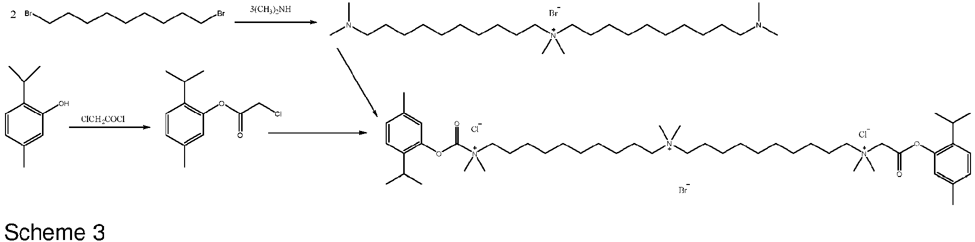

### Example 4

### Synthesis of the compound of formula (VII)

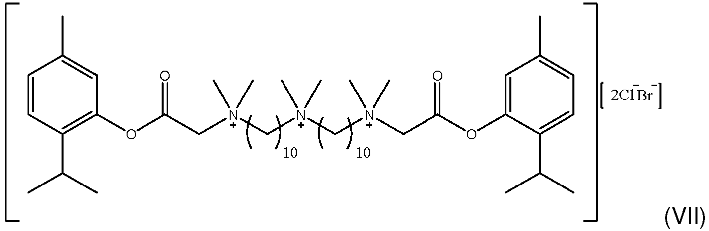

In a first step, 2 molar equivalents of 1,10-Dibromodecane are reacted with 3 molar equivalents of dimethylamine The reaction takes place at 4-5° C in a suitable solvent such as benzene or diethyl ether and is followed by a step of acid extraction followed by alkaline treatment and extraction with diethyl ether. The extracted fractions are dried over magnesium sulphate and then purified by vacuum distillation.

In a second step, thymol (2-isopropyl-5-methylphenol) is reacted with chloroacetyl chloride. The reaction is carried out at -10 C for 1 hour and then stirred at room temperature for 12 hours. The reaction mixture is then washed with acid, followed by treatment with sodium bicarbonate and then water. The organic layer is dried over sodium sulphate, filtered and the solvent is removed under vacuum.

In a third step, one molar equivalent of the compound formed in step 1 is reacted with the 2 molar equivalents of the compound formed in step 2 to form the compound having formula (VII). The reaction in the third step is carried out using an appropriate solvent such as acetonitrile and the reaction mixture is stirred for 24 hours.

It will be appreciated that further purification and separation steps may also be included in the process, for example between each of the above steps and also after the process is complete to purify the final compound having formula (VII).

Separation steps may include steps of performing column chromatography, low pressure liquid chromatography, high performance liquid chromatography and the like. Purification steps may include standard purification processes known in the art, for example, filtration, evaporation, liquid-liquid extraction, crystallisation, adsorption, recrystallization, chromatography, distillation and the like.

| | |
|---|---|
| Systematic Name: | N1-(2-(2-isopropyl-5-methylphenoxy)-2-oxoethyl)-N10-(10-((2-(2-isopropyl-5-methylphenoxy)-2-oxoethyl)dimethylammonio)decyl)- |
| | N1,N1,N10,N10-tetramethyldecane-1,10-diaminium bromide dichloride |
| Formula: | C₅₀H₈₈N₃O₄⁺³ Br⁻2Cl⁻ |
| Molecular Weight: | 946.06 |

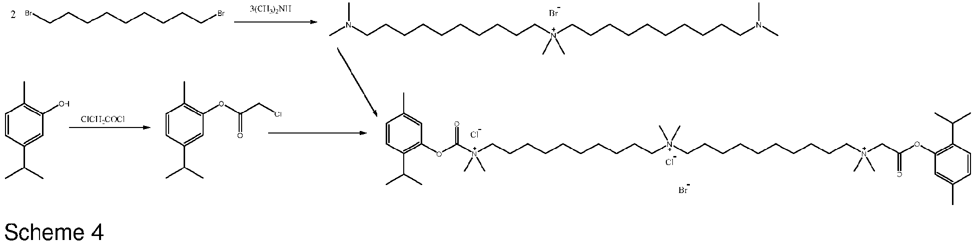

It will be appreciated that to synthesise the bromine complexes defined by formula XI to XIV, the compounds of formula IV to VII are treated with bromine to form the complexes of formulas XI to XIV. It will be appreciated that further purification and separation steps may also be included in this process.

### Example 5 - Evaluation of inhibition of Sars-Cov-2 reporter gene by compound IV

Compound IV was evaluated using a SARS-COV-2 reporter gene assay which uses a reporter gene tagged infectious SARS-CoV-2 virus. The assay is described in the following article: Xie et al.; bioRxiv . 2020 Jun 23;2020.06.22.165712; doi: 10.1101/2020.06.22.165712.

The following SARS-Cov-2 nanoluciferase (SARS-Cov-2 Nluc) antiviral assay protocol was used.

A549-hACE2 cells (12,000 cells per well in phenol-red free medium containing 2% fetal bovine serum (FBS)) were plated into a white opaque 96-well plate (Corning).

3-fold serial dilutions from 10µM (final assay concentration) of compound IV were prepared in water in 96 well format and further diluted 100-fold in the phenol-red free culture medium containing 2% FBS.

Cell culture medium was removed and incubated with 50 µL of the diluted compound solutions and 50 µL of SARS-CoV2-Nluc viruses (at Multiplicity of Infection (MOI) of 0.025).

At 48 h post-infection, 50 µL Nano luciferase substrates (Promega) were added to each well and Luciferase signals measured using a Synergy^{™} Neo2 microplate reader

Relative luciferase signals were calculated by normalizing the luciferase signals of compound-treated groups to that of the water-treated groups (set as 100%)

The EC50 (compound concentration for reducing 50% of luciferase signal) data were calculated from technical repeats using a nonlinear regression model

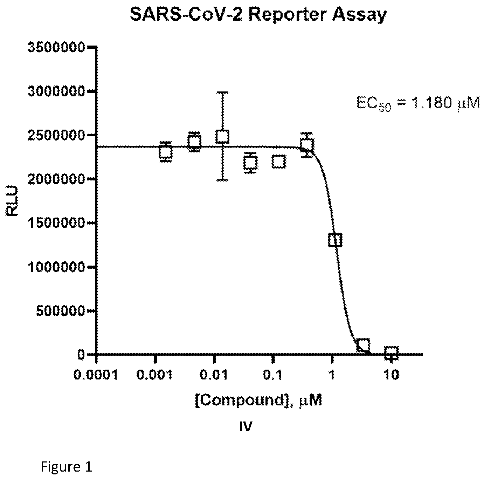

The results of the assay are illustrated in figure 1.

Figure 1 shows the results of the assay and indicates that compound IV inhibits the SARS-CoV-2 reporter gene in a dose dependent manner.

The effect of compound IV on cell viability (CC50 value) was determined using the Cell TiterGlo (CTG) assay protocol (Promega) in a similar fashion except that the virus infection was omitted.

Compound IV was tested using the CellTiter-Glo^{®} Luminescent Cell Viability Assay.

The assay principle of the homogeneous CellTiter-Glo^{®} Luminescent Cell Viability Assay is based on determining the number of viable cells in tissue culture and dependent on the quantification of the ATP content correlating with the number of metabolically active cells (www.promega.sg). Following the instructions of the manufacturer, the assay is used in multi-well plate formats for determination of EC50 values of test compounds in standard readouts such as cell proliferation and cytotoxicity. For the evaluation of Compound IV, A549 NSCLC cells were seeded at 12,000 cells per well in 96 well plate format. 12 hours after seeding, the test compound was added to the cells and treatment continued for 48h. In order to evaluate Compound IV over a broad concentration range starting from 10µM as the highest concentration, a 3-fold serial dilution was conducted with a total of 10 data points. At the end of the treatment, cells were processed according to the manufacturer's instructions and luminescence was measured using a Tecan plate reader M1000. The EC50 value of Compound IV was determined using the GraphPad Prism software.

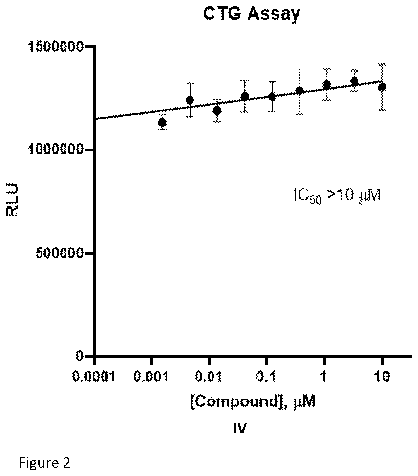

The results of this assay are illustrated in Figure 2, which indicates that compound IV does not affect cell viability.

**Table 1**

| **Compound** | **Structure** |
|---|---|
| **Compound IV** | 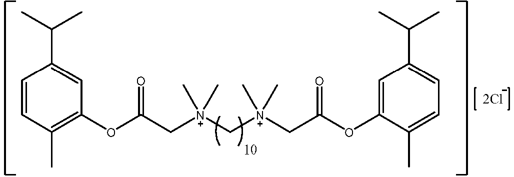 |

### Example 6 - anti-African Swine Fever properties of compounds IV and V

### Experimental

RPMI media was prepared at 37ºC and glutamine and HEPES (4-(2-hydroxyethyl)-1-piperazineethanesulfonic acid) buffer were added to the media.

0.1 grams of each of the tested compounds (compounds IV and V) was dissolved separately in 3 mL distilled water (pH 5).

2.5mL of media was transferred to a first tube and 2.5mL of the test compound (Compound IV or V) solution was added to the tube. 2.5 mL of the mixture was then transferred to a second tube and 2.5mL of RPMI media was added to the second tube to form a diluted mixture. This 2 fold dilution step was repeated sequentially until 16 tubes were filled.

100 µL of each diluted sample (1-16) was transferred from each tube and placed in a 42-well microtiter plate and mixed with 100 µL of log 5 pure African swine fever virus solution to form a mixture. Each mixture was incubated for 3 hours.

For the negative control samples, 200 µL of RPMI media was placed in the wells. For the positive control group, 100 µL of RPMI media was mixed with 100 µL of the virus solution and transferred to the wells.

Each incubated mixture was then transferred to the primary cells (PAM cells) and viral multiplication and cell toxicity were observed at 0, 24, 48, 72 and 96 hours post infection. Viral multiplication was determined by the observation of rosetta on the surface of the cell plates.

**Table 2a: Compound IV dilution procedure.**

| **Sample** | **1** | **2** | **3** | **4** | **5** | **6** | **7** | **8** | **9** | **10** | **11** | **12** |
|---|---|---|---|---|---|---|---|---|---|---|---|---|
| Compound IV (mL) | 2.5 | 1.25 | 0.625 | 0.3125 | 0.156 | 0.08 | 0.04 | 0.02 | 0.01 | 0.005 | 0.0025 | 0.00125 |
| RPMI (mL) | 2.5 | 3.75 | 4.375 | 4.6875 | 4.998 | 4.92 | 4.96 | 4.98 | 4.99 | 4.995 | 4.9975 | 4.99875 |
| Compound IV (%) | 25% | 13% | 6.3% | 3.1% | 1.56% | 0.8% | 0.4% | 0.2% | 0.1% | 0.05% | 0.025% | 0.0125% |

**Table 2b: Compound IV dilution procedure.**

| **Sample** | **13** | **14** | **15** | **16** | **Positive Control** | **Negative Control** |
|---|---|---|---|---|---|---|
| Compound IV (mL) | 0.000625 | 0.000312 | 0.000156 | 0.000078 | - | - |
| RPMI (mL) | 4.999375 | 4.999688 | 4.999844 | 4.999921 | 5 | 5 |
| Compound IV (%) | 0.0063% | 0.0032% | 0.0016% | 0.0008% | - | - |

**Table 3a: Compound V dilution procedure.**

| **Sample** | **1** | **2** | **3** | **4** | **5** | **6** | **7** | **8** | **9** | **10** | **11** | **12** |
|---|---|---|---|---|---|---|---|---|---|---|---|---|
| Compound V (mL) | 2.5 | 1.25 | 0.625 | 0.3125 | 0.156 | 0.08 | 0.04 | 0.02 | 0.01 | 0.005 | 0.0025 | 0.00125 |
| RPMI (mL) | 2.5 | 3.75 | 4.375 | 4.6875 | 4.998 | 4.92 | 4.96 | 4.98 | 4.99 | 4.995 | 4.9975 | 4.99875 |
| Compound V (%) | 25% | 13% | 6.3% | 3.1% | 1.56% | 0.8% | 0.4% | 0.2% | 0.1% | 0.05% | 0.025% | 0.0125% |

**Table 3b: Compound V dilution procedure.**

| **Sample** | **13** | **14** | **15** | **16** | **Positive Control** | **Negative Control** |
|---|---|---|---|---|---|---|
| Compound V (mL) | 0.000625 | 0.000312 | 0.000156 | 0.000078 | - | - |
| RPMI (mL) | 4.999375 | 4.999688 | 4.999844 | 4.999921 | 5 | 5 |
| Compound V (%) | 0.0063% | 0.0032% | 0.0016% | 0.0008% | - | - |

### Results

The results for both compounds IV and V are described in table 4 below.

**Table 4**

| **Sample** | **Result** |
|---|---|
| **Positive control** | African swine fever infected cells. Presence of a large number of rosetta was observed. |
| **1-9** | Cells did not grow. Compounds IV and V formed a layer around the cells, inhibiting growth. |
| **10-13** | Cells grew well and were protected from African swine fever virus infection. No rosetta were observed. |
| 14-16 | Cells grew well, but protection against ASFV infection decreased and some rosetta became visible. |
| **Negative control** | Cells grew well |

As demonstrated in Table 4, Compounds IV and V were both effective at inhibiting African swine fever virus.

**Table 5**

| **Compound** | **Structure** |
|---|---|
| **Formula Compound IV** | 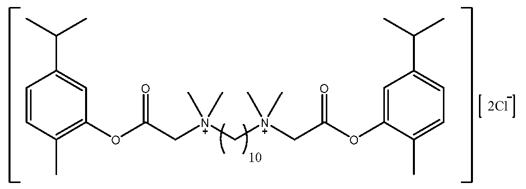 |
| **Compound V** | 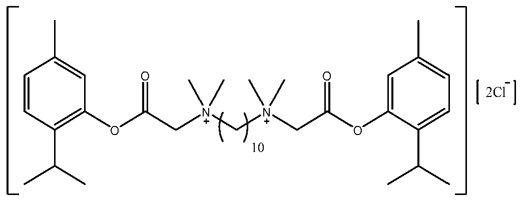 |

## Claims

1. A compound for use in the treatment of an infection caused by a coronavirus or African Swine fever virus, wherein the compound has formula I: 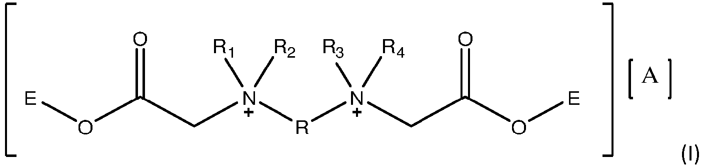
wherein R is an alkylene chain having between 8 and 20 carbon atoms, and A is one or more anions having a total charge of -2,
or R is a group having the following formula: 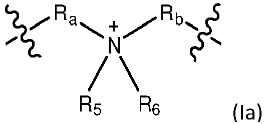
wherein Rₐ and R_{b} are each an alkylene chain having between 8 and 20 carbon atoms, and A is one or more anions having a total charge of -3;
R₁, R₂, R₃, R₄, R₅ and R₆ are each independently selected from C₁₋₁₀ alkyl and H;
E has the following formula 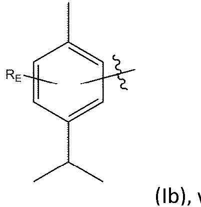 wherein the ester oxygen is bonded to the aromatic ring of each E at the same position, the position being position 2 or 3; and wherein R_{E} is H or a halide.

2. The compound for use according to claim 1, wherein the infection is caused by a coronavirus, for example severe acute respiratory syndrome coronavirus for example wherein the infection is caused by Severe acute respiratory syndrome coronavirus 2 (SARS-CoV-2).

3. The compound for use according to any preceding claim, wherein the compound is for use in treating a coronavirus disease, for example wherein the compound is for use in treating coronavirus disease 2019 (COVID-19).

4. The compound for use according to claim 1, wherein the infection is caused by African Swine fever virus and/or wherein the compound is for use in treating African Swine fever.

5. The compound for use according to any preceding claim, wherein the compound of formula I has the following formula: 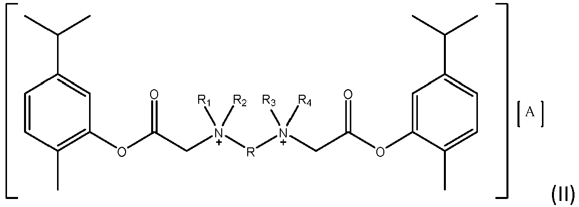 or

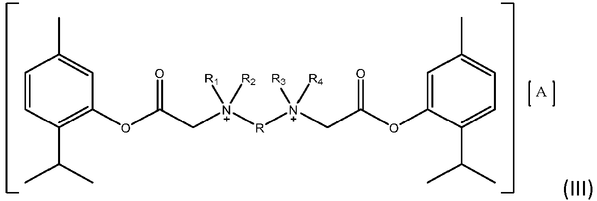

6. The compound for use according to claim 5, wherein R is a saturated linear alkylene chain having between 8 and 16 carbon atoms, for example 10 carbon atoms; and/or wherein Rₐ and R_{b} are each a saturated linear alkylene chain having between 8 and 16 carbon atoms, for example 10 carbon atoms and/or wherein the one or more anions are selected from chloride anions and bromide anions and/or wherein R₁, R₂, R₃, R₄, R₅ and R₆ are each methyl.

7. The compound for use according to claim 1, wherein the compound of formula I has the following formulae: 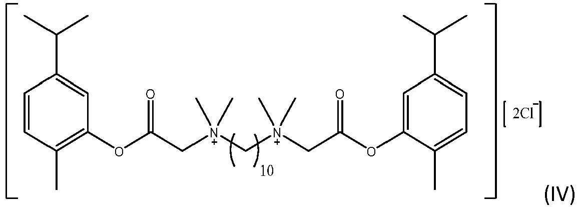 or 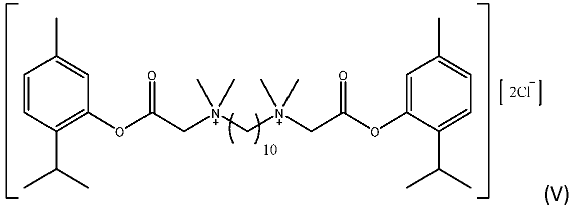 or 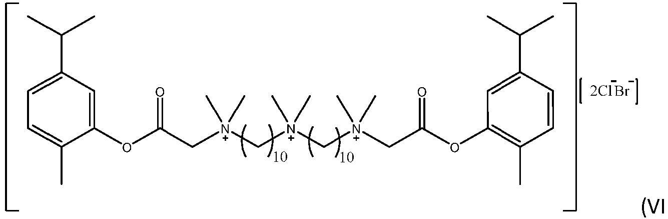 or

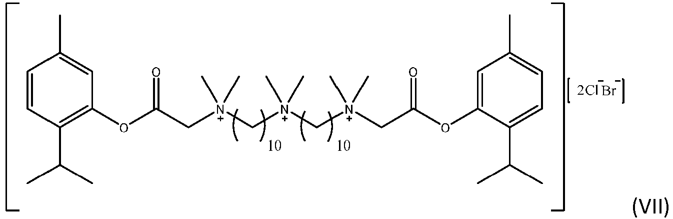

8. The compound for use according to claim 2 or 3, wherein the compound of formula I has the following formula: 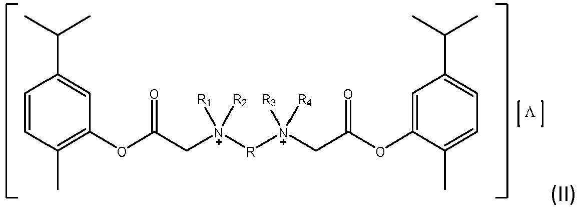 wherein R is an alkylene chain having between 8 and 20 carbon atoms; for example wherein R is a saturated linear alkylene chain having between 8 and 16 carbon atoms, for example 10 carbon atoms.

9. The compound for use according to claim 8, wherein the compound of formula II has the following formula:

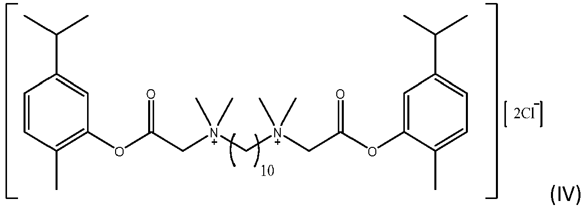

10. The compound for use according to claim 4, wherein the compound of formula (I) has the following formula: 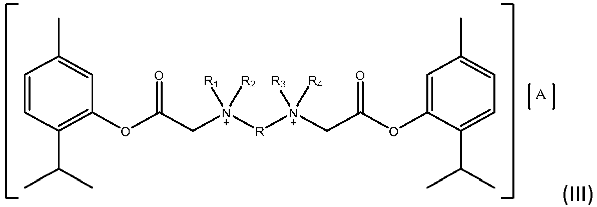 or 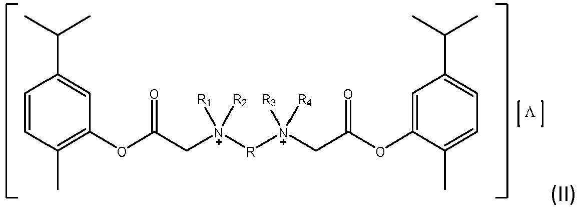 wherein R is an alkylene chain having between 8 and 20 carbon atoms; for example wherein R is a saturated linear alkylene chain having between 8 and 16 carbon atoms, for example 10 carbon atoms.

11. The compound for use according to claim 10, wherein the compound of formula III has the following formula: 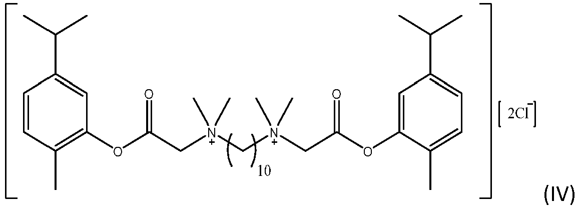 or wherein the compound of formula II has the following formula:

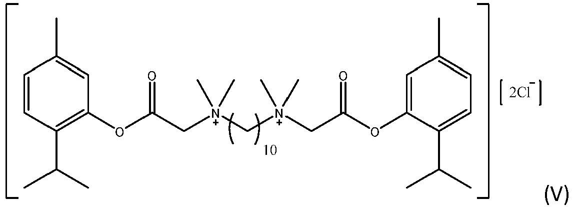

12. A compound for use according to any one of claims 1 to 4, wherein the compound has formula VIII: 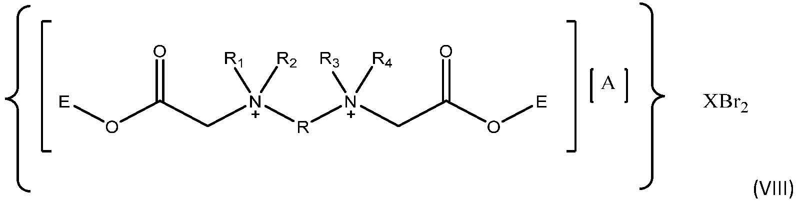
wherein R is an alkylene chain having between 8 and 20 carbon atoms, and A is one or more anions having a total charge of -2,
or R is a group having the following formula: 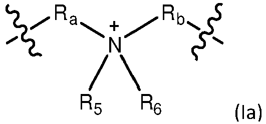
wherein Rₐ and R_{b} are each an alkylene chain having between 8 and 20 carbon atoms, and A is one or more anions having a total charge of -3;
R₁, R₂, R₃, R₄, R₅ and R₆ are each independently selected from C₁₋₁₀ alkyl and H;
E has the following formula 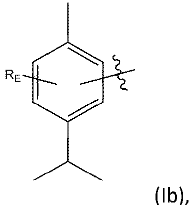
wherein the ester oxygen is bonded to the aromatic ring of each E at the same position, the position being 2 or 3; wherein R_{E} is H or a halide; and wherein X is 2, 3, 4 or 6 for example, wherein the compound of formula VIII has the following formula: 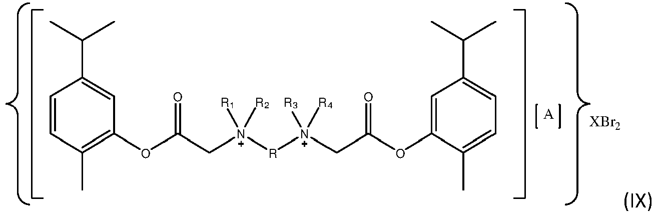 or

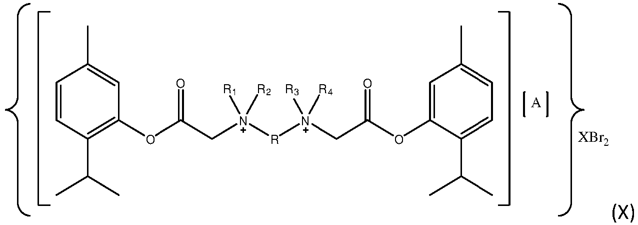

13. A compound for use according to claim 12, wherein R is a saturated linear alkylene chain having between 8 and 16 carbon atoms, for example 10 carbon atoms; or wherein Rₐ and R_{b} are each a saturated linear alkylene chain having between 8 and 16 carbon atoms, for example 10 carbon atoms.

14. A compound for use according to claim 12, wherein the one or more anions are selected from chloride anions and bromide anions.

15. A compound for use according to claim 12, wherein the compound has the following formula: 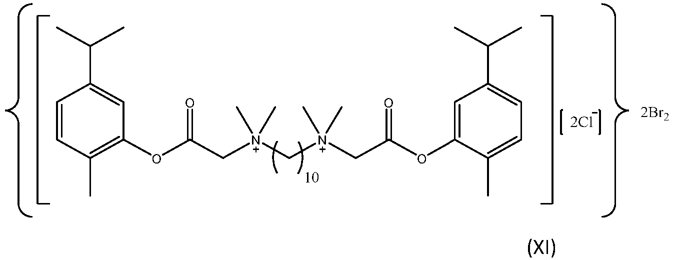 or 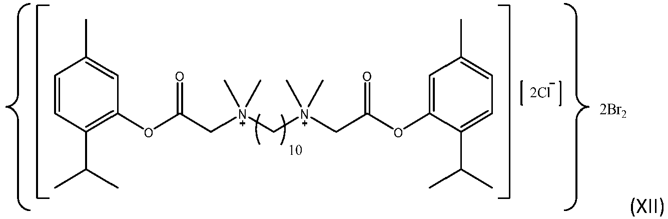 or 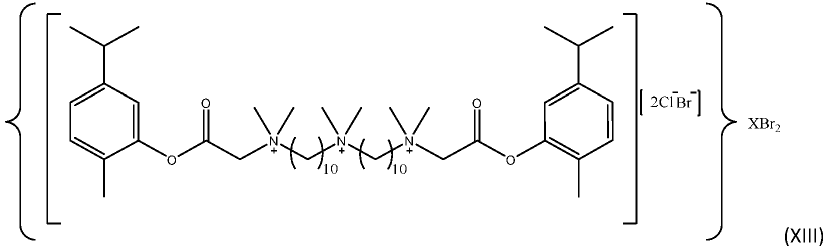 or or 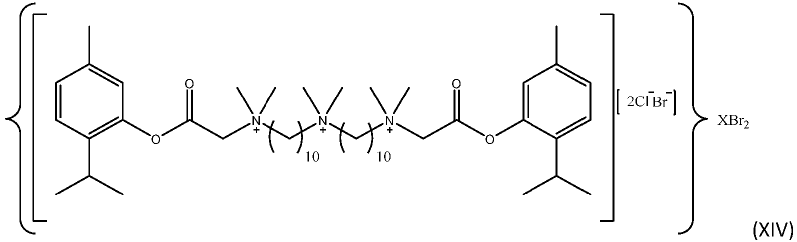 wherein X is 3 or 6.

## Patentansprüche

1. Verbindung zur Verwendung bei der Behandlung einer durch ein Coronavirus oder ein afrikanisches Schweinepestvirus verursachten Infektion, wobei die Verbindung Formel I hat: 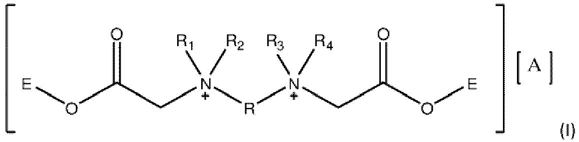
wobei R eine Alkylenkette mit 8 bis 20 Kohlenstoffatomen ist und A ein oder mehrere Anionen mit einer Gesamtladung von -2 ist, oder
R eine Gruppe mit der folgenden Formel ist: 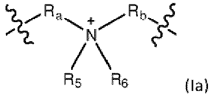 wobei Rₐ und R_{b} jeweils eine Alkylenkette mit 8 bis 20 Kohlenstoffatomen sind und A ein oder mehrere Anionen mit einer Gesamtladung von -3 ist;
R₁, R₂, R₃, R₄, R₅ und R₆ jeweils unabhängig aus C₁₋₁₀-Alkyl und H ausgewählt sind;
E die Formel 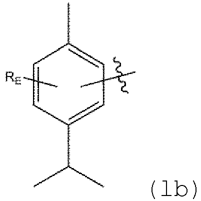 hat, wobei der Ester-Sauerstoff an den aromatischen Ring jedes E an derselben Position gebunden ist, wobei die Position die Position 2 oder 3 ist; und wobei R_{E} H oder ein Halogenid ist.

2. Verbindung zur Verwendung nach Anspruch 1, wobei die Infektion durch ein Coronavirus verursacht wird, zum Beispiel durch das schwere akute Atemwegssyndrom-Coronavirus, zum Beispiel wobei die Infektion durch das schwere akute Atemwegssyndrom-Coronavirus 2 (SARS-CoV-2) verursacht wird.

3. Verbindung zur Verwendung nach einem vorherigen Anspruch, wobei die Verbindung zur Verwendung bei der Behandlung einer Coronavirus-Erkrankung bestimmt ist, wobei die Verbindung zum Beispiel zur Verwendung bei der Behandlung der Coronavirus-Erkrankung 2019 (COVID-19) bestimmt ist.

4. Verbindung zur Verwendung nach Anspruch 1, wobei die Infektion durch das afrikanische Schweinepestvirus verursacht wird und/oder wobei die Verbindung zur Verwendung bei der Behandlung der afrikanischen Schweinepest bestimmt ist.

5. Verbindung zur Verwendung nach einem vorherigen Anspruch, wobei die Verbindung von Formel I die folgende Formel hat: 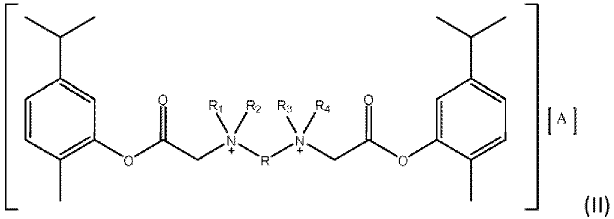 oder

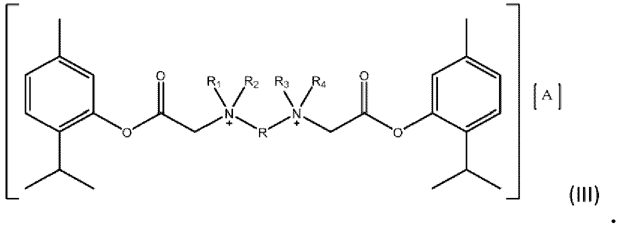

6. Verbindung zur Verwendung nach Anspruch 5, wobei R eine gesättigte lineare Alkylenkette mit 8 bis 16 Kohlenstoffatomen, beispielsweise 10 Kohlenstoffatomen, ist; und/oder wobei Rₐ und R_{b} jeweils eine gesättigte lineare Alkylenkette mit 8 bis 16 Kohlenstoffatomen, beispielsweise 10 Kohlenstoffatomen, sind, und/oder wobei die ein oder mehreren Anionen aus Chloridanionen und Bromidanionen ausgewählt sind, und/oder wobei R₁, R₂, R₃, R₄, R₅ und R₆ jeweils Methyl sind.

7. Verbindung zur Verwendung nach Anspruch 1, wobei die Verbindung von Formel I die folgenden Formeln hat: 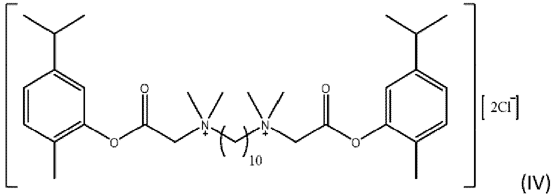 oder 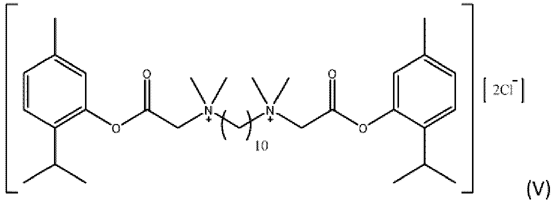 oder 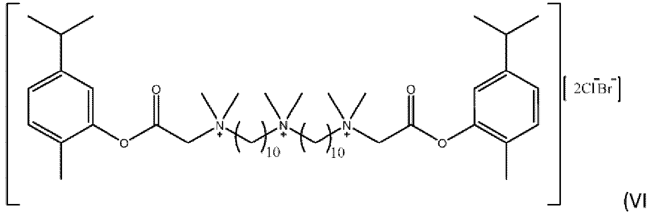 oder

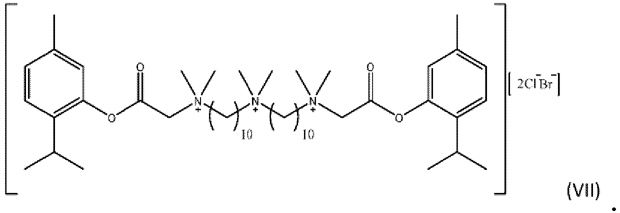

8. Verbindung zur Verwendung nach Anspruch 2 oder 3, wobei die Verbindung von Formel I die folgende Formel hat: 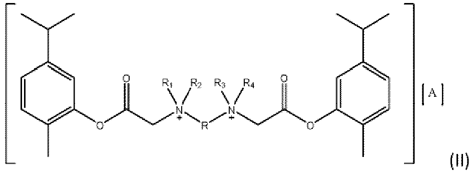 wobei R eine Alkylenkette mit 8 bis 20 Kohlenstoffatomen ist; wobei R beispielsweise eine gesättigte lineare Alkylenkette mit 8 bis 16 Kohlenstoffatomen, beispielsweise 10 Kohlenstoffatomen, ist.

9. Verbindung zur Verwendung nach Anspruch 8, wobei die Verbindung von Formel II die folgende Formel hat:

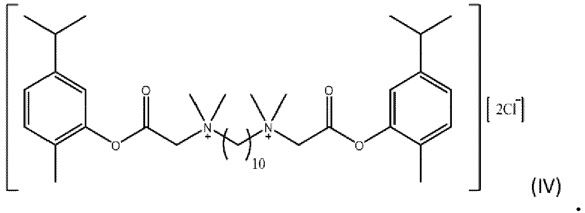

10. Verbindung zur Verwendung nach Anspruch 4, wobei die Verbindung von Formel (I) die folgende Formel hat: 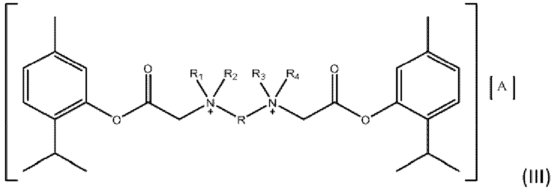 oder 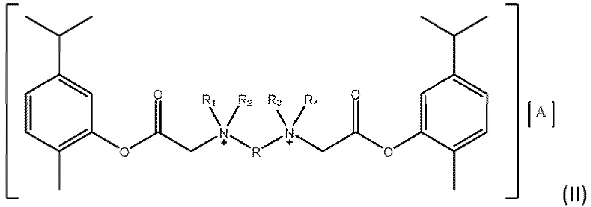 wobei R eine Alkylenkette mit 8 bis 20 Kohlenstoffatomen ist; wobei R beispielsweise eine gesättigte lineare Alkylenkette mit 8 bis 16 Kohlenstoffatomen, beispielsweise 10 Kohlenstoffatomen, ist.

11. Verbindung zur Verwendung nach Anspruch 10, wobei die Verbindung von Formel III die folgende Formel hat: 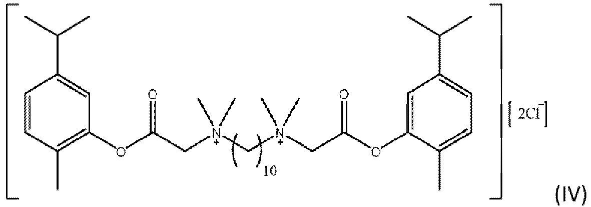 oder wobei die Verbindung von Formel II die folgende Formel hat:

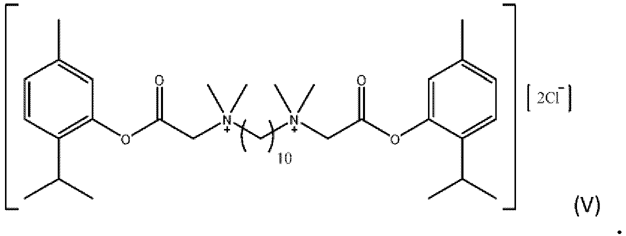

12. Verbindung zur Verwendung nach einem der Ansprüche 1 bis 4, wobei die Verbindung Formel VIII hat: 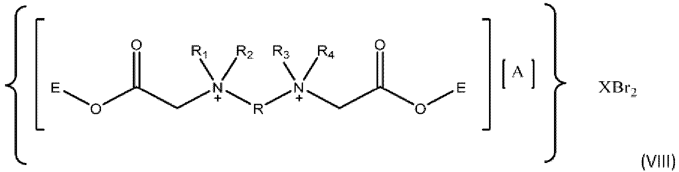
wobei R eine Alkylenkette mit 8 bis 20 Kohlenstoffatomen ist und A ein oder mehrere Anionen mit einer Gesamtladung von -2 ist, oder
R eine Gruppe der folgenden Formel ist: 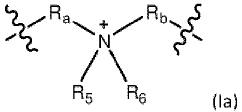
wobei Rₐ und R_{b} jeweils eine Alkylenkette mit 8 bis 20 Kohlenstoffatomen sind und A ein oder mehrere Anionen mit einer Gesamtladung von -3 ist;
R₁, R₂, R₃, R₄, R₅ und R₆ jeweils unabhängig aus C₁₋₁₀ Alkyl und H ausgewählt sind;
E die folgende Formel 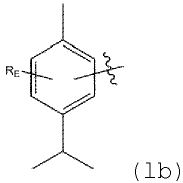 hat, wobei der Ester-Sauerstoff an den aromatischen Ring jedes E an der gleichen Position gebunden ist, wobei die Position 2 oder 3 ist; wobei R_{E} H oder ein Halogenid ist; und
wobei X beispielsweise 2, 3, 4 oder 6 ist, wobei die Verbindung von Formel VIII die folgende Formel hat: 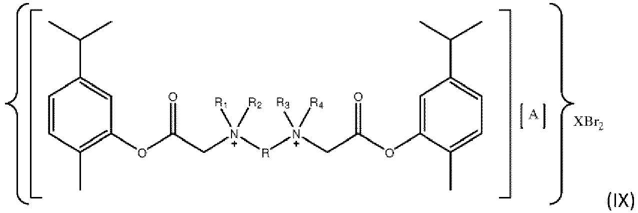 oder

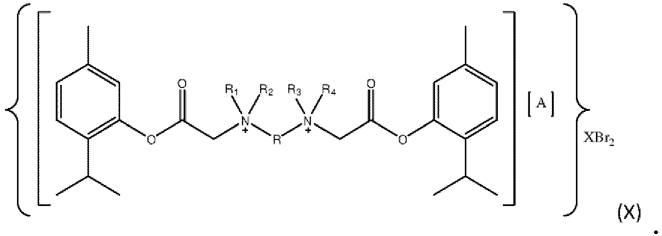

13. Verbindung zur Verwendung nach Anspruch 12, wobei R eine gesättigte lineare Alkylenkette mit 8 bis 16 Kohlenstoffatomen, beispielsweise 10 Kohlenstoffatomen, ist; oder wobei Rₐ und R_{b} jeweils eine gesättigte lineare Alkylenkette mit 8 bis 16 Kohlenstoffatomen, beispielsweise 10 Kohlenstoffatomen, sind.

14. Verbindung zur Verwendung nach Anspruch 12, wobei die ein oder mehreren Anionen aus Chloridanionen und Bromidanionen ausgewählt sind.

15. Verbindung zur Verwendung nach Anspruch 12, wobei die Verbindung die folgende Formel hat: 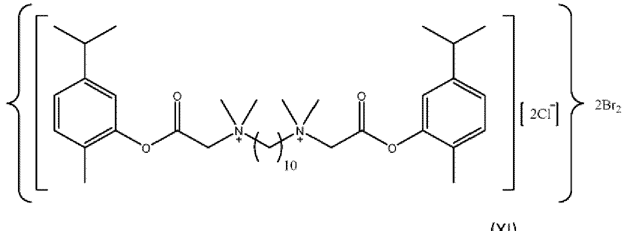 oder 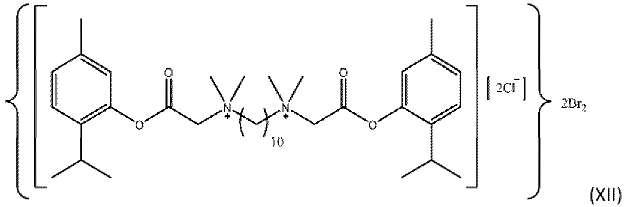 oder 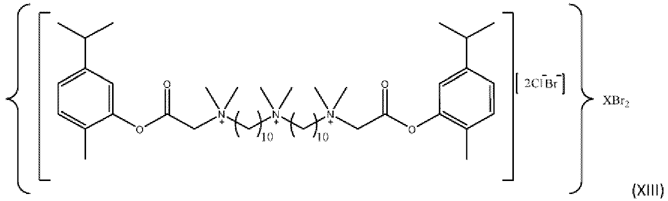 oder 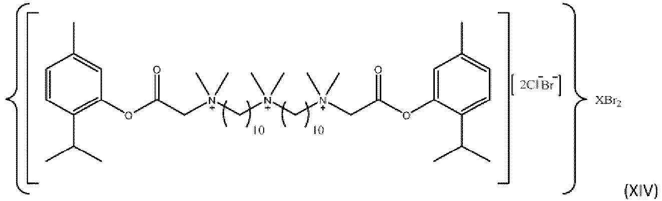 wobei X 3 oder 6 ist.

## Revendications

1. Composé destiné à être utilisé dans le traitement d'une infection causée par un coronavirus ou un virus de la peste porcine africaine, le composé ayant la formule I : 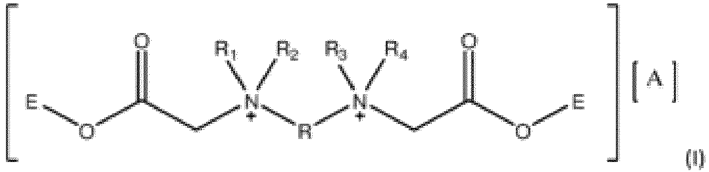
dans lequel R est une chaîne alkylène ayant entre 8 et 20 atomes de carbone, et A est un ou plusieurs anions ayant une charge totale de -2,
dans lequel R est un groupe de la formule suivante : 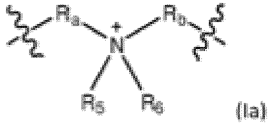
dans lequel Rₐ et R_{b} sont chacun une chaîne alkylène ayant entre 8 et 20 atomes de carbone, et A est un ou plusieurs anions ayant une charge totale de -3 ;
R₁, R₂, R₃, R₄, R₅ et R₆ sont chacun sélectionnés indépendamment parmi un alkyle en C₁₋₁₀ et H ;
E a la formule suivante 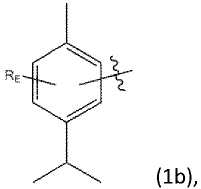 où l'ester d'oxygène est lié au cycle aromatique de chaque E à la même position, la position étant la position 2 ou 3 ; et où R_{E} est H ou un halogénure.

2. Composé destiné à être utilisé selon la revendication 1, dans lequel l'infection est causée par un coronavirus, par exemple un coronavirus de syndrome respiratoire aigu sévère, par exemple dans lequel l'infection est causée par le coronavirus de syndrome respiratoire aigu sévère 2 (SARS-CoV-2).

3. Composé destiné à être utilisé selon l'une quelconque des revendications précédentes, dans lequel le composé est destiné à être utilisé dans le traitement d'une maladie à coronavirus, par exemple dans lequel le composé est destiné à être utilisé dans le traitement de la maladie à coronavirus 2019 (COVID-19).

4. Composé destiné à être utilisé selon la revendication 1, dans lequel l'infection est causée par le virus de la peste porcine africaine et/ou dans lequel le composé est destiné à être utilisé dans le traitement de la peste porcine africaine.

5. Composé destiné à être utilisé selon l'une quelconque des revendications précédentes, dans lequel le composé de formule I a la formule suivante : 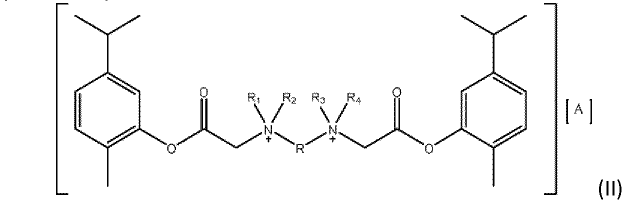 ou

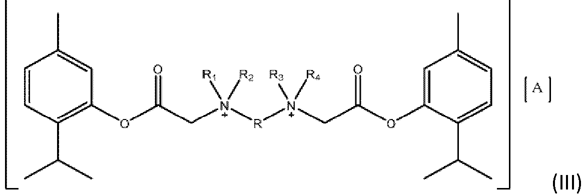

6. Composé destiné à être utilisé selon la revendication 5, dans lequel R est une chaîne alkylène linéaire saturée ayant entre 8 et 16 atomes de carbone, par exemple 10 atomes de carbone ; et/ou dans lequel Rₐ et R_{b} sont chacun une chaîne alkylène linéaire saturée ayant entre 8 et 16 atomes de carbone, par exemple 10 atomes de carbone et/ou dans lequel les un ou plusieurs anions sont sélectionnés parmi les anions chlorure et les anions bromure et/ou dans lequel R₁, R₂, R₃, R₄, R₅ et R₆ sont chacun un méthyle.

7. Composé destiné à être utilisé selon la revendication 1, dans lequel le composé de formule I a les formules suivantes : 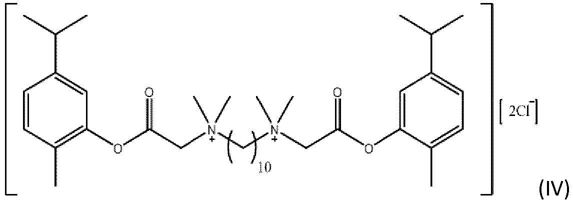 ou

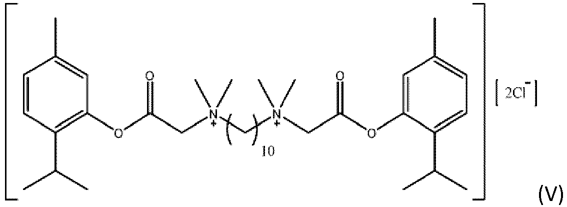

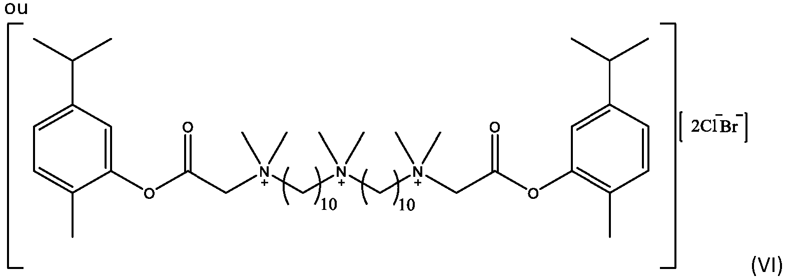

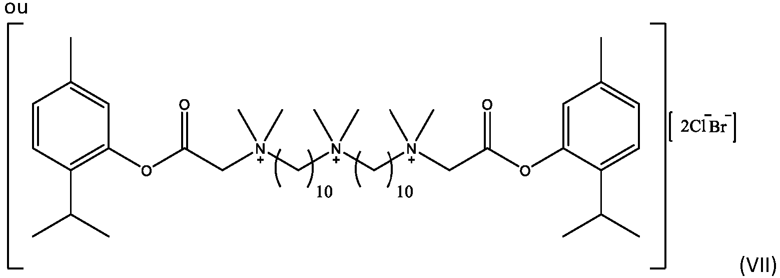

8. Composé destiné à être utilisé selon la revendication 2 ou 3, dans lequel le composé de formule I a la formule suivante : 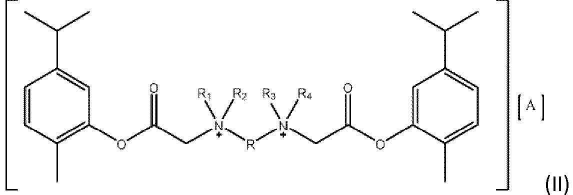 dans lequel R est une chaîne alkylène ayant entre 8 et 20 atomes de carbone ; par exemple, dans lequel R est une chaîne alkylène linéaire saturée ayant entre 8 et 16 atomes de carbone, par exemple 10 atomes de carbone.

9. Composé à utiliser selon la revendication 8, dans lequel le composé de formule Il a la formule suivante :

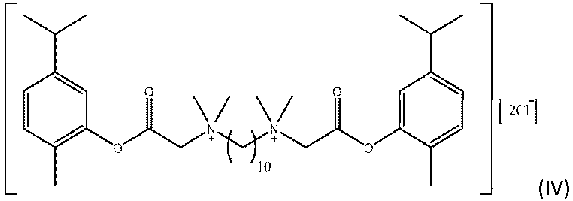

10. Composé destiné à être utilisé selon la revendication 4, dans lequel le composé de formule (I) a la formule suivante : 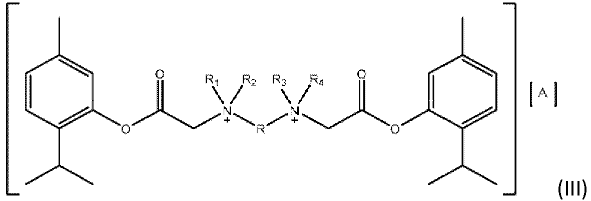 ou 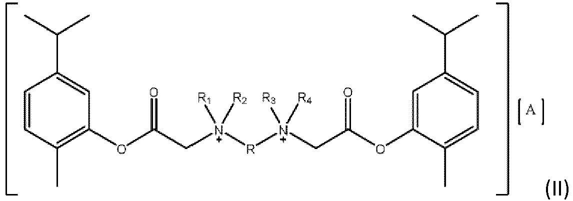 dans lequel R est une chaîne alkylène ayant entre 8 et 20 atomes de carbone ; par exemple, dans lequel R est une chaîne alkylène linéaire saturée ayant entre 8 et 16 atomes de carbone, par exemple 10 atomes de carbone.

11. Composé destiné à être utilisé selon la revendication 10, dans lequel le composé de formule III a la formule suivante : 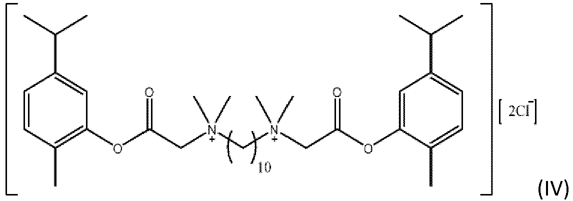 ou dans lequel le composé de formule Il a la formule suivante :

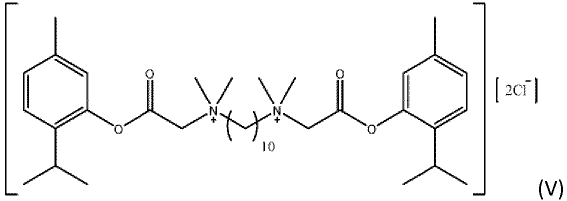

12. Composé destiné à être utilisé selon l'une quelconque des revendications 1 à 4, dans lequel le composé a la formule VIII : 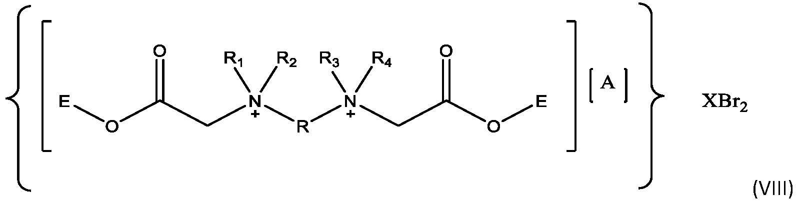
dans lequel R est une chaîne alkylène ayant entre 8 et 20 atomes de carbone, et A est un ou plusieurs anions ayant une charge totale de -2,
ou R est un groupe de la formule suivante : 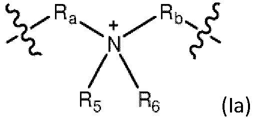
dans lequel Rₐ et R_{b} sont chacun une chaîne alkylène ayant entre 8 et 20 atomes de carbone, et A est un ou plusieurs anions ayant une charge totale de -3 ;
R₁, R₂, R₃, R₄, R₅ et R₆ sont chacun sélectionnés indépendamment parmi un alkyle en C₁₋₁₀ et H ;
E a la formule suivante 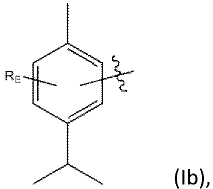 dans lequel l'ester d'oxygène est lié au cycle aromatique de chaque E à la même position, la position étant 2 ou 3 ; dans lequel R_{E} est H ou un halogénure ; et dans lequel X est 2, 3, 4 ou 6 par exemple, dans lequel le composé de formule VIII a la formule suivante : 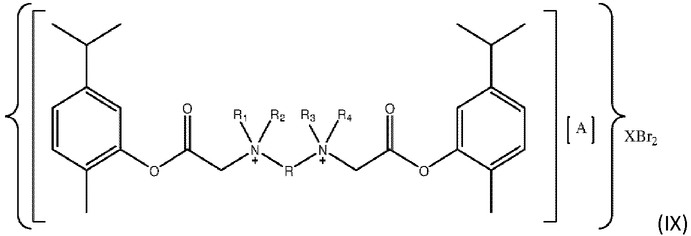 Ou

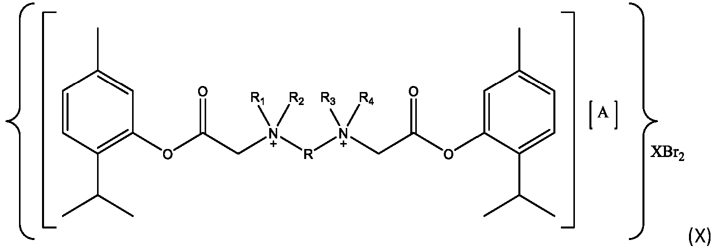

13. Composé destiné à être utilisé selon la revendication 12, dans lequel R est une chaîne alkylène linéaire saturée ayant entre 8 et 16 atomes de carbone, par exemple 10 atomes de carbone ; ou dans lequel Rₐ et R_{b} sont chacun une chaîne alkylène linéaire saturée ayant entre 8 et 16 atomes de carbone, par exemple 10 atomes de carbone.

14. Composé destiné à être utilisé selon la revendication 12, dans lequel les un ou plusieurs anions sont sélectionnés parmi les anions chlorure et les anions bromure.

15. Composé destiné à être utilisé selon la revendication 12, le composé ayant la formule suivante : 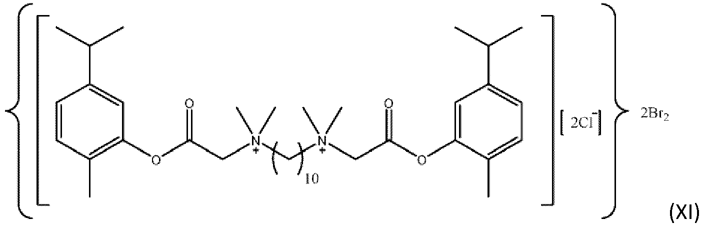 ou 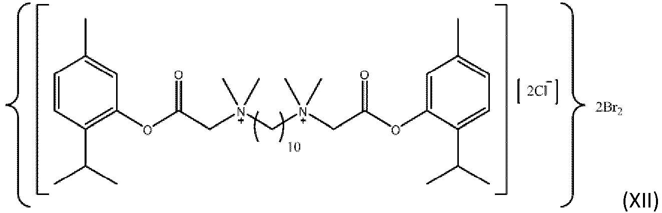 ou 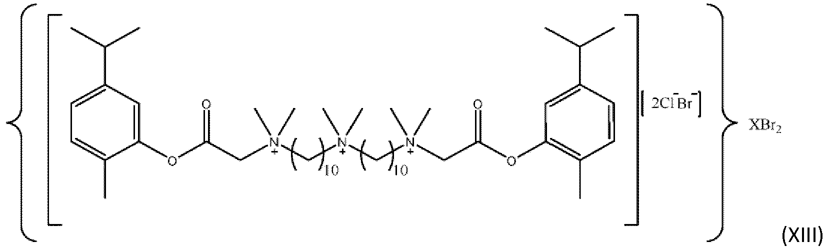 ou 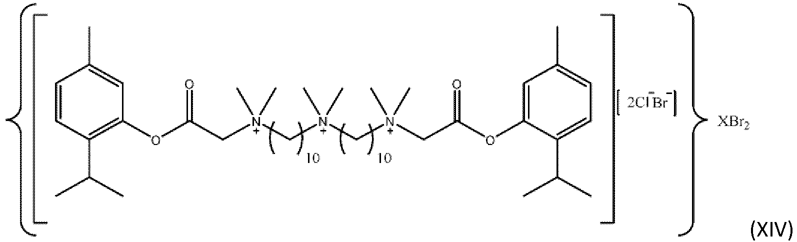 ou
dans lequel X est 3 ou 6.
